# EUROPEAN PATENT APPLICATION

(11) **EP 1 452 177 A1**
(43) Date of publication of application: **01.09.2004**
(21) Application number: 03004191.7
(22) Date of filing: 27.02.2003
(51) Int. Cl.: A61K 31/55, A61K 47/20

(54) **Pharmaceutical formulations comprising sodium laurylsulfate as bitterness masking agent**

(71) Applicant: Boehringer Ingelheim International GmbH, 55216 Ingelheim am Rhein (DE)
(72) Inventor: Fujita, Hikaru, Osaka-city, Osaka-pref. (JP); Kuroki, Akiko, Takarazuka-city, Hyogo-pref. (JP)

(57) **Abstract**

The present invention refers to a new bitterness masking agent for pharmaceutical formulations which are to be applied orally. It was found that certain pharmaceutically acceptable surfactants can mask the bitter taste of some bitter tasting pharmaceutically active compounds. The present invention in particular concerns epinastine or quinine comprising formulations.

## Description

The present invention refers to a new bitterness masking agent for pharmaceutical formulations which are to be applied orally. It was found that certain pharmaceutically acceptable surfactants can mask the bitter taste of some bitter tasting pharmaceutically active compounds. The present invention in particular concerns epinastine or quinine comprising formulations.

### State of the Art

If pharmaceutical compositions have a bitter taste they may be mixed with a sweetening agent to mask the bitter taste. Among many pharmaceutical active substances which have a bitter taste are epinastine and quinine.

Epinastine, chemically known as 3-Amino-9,13b-dihydro-1H-dibenz-[c,f]imidazol[1,5-a]azepine and its acid addition salts are disclosed for the first time in the German Patent application P 30 08 944.2 which forms the basis for EP 0035749.

Chemically epinastine is an amino base and represented by the following formula, which does not reflect stereochemical properties:

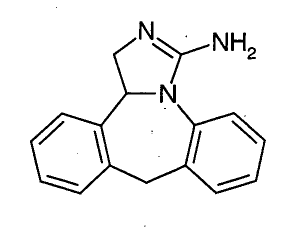

Epinastine can be used either as free base or as a pharmaceutically acceptable salt thereof. Preferably, epinastine is used as hydrochloride.

If not specified further in the context of the present invention the term epinastine is used for the free base as well as for pharmaceutically acceptable salts.

Methods for its preparations can be taken from EP 0496306 or from WO 01/40229.

Epinastine among others is for treating allergies, pain, in particular chronic pain and inflammation caused pain, migraine, asthma, rhinitis, conjunctivitis and/or bronchitis (f.e. EP-B-0 035 749, EP 1000623). In particular, epinastine is for treating allergies, dermatitis, rhinitis, conjunctivitis, bronchitis and asthma.

Epinastine is marketed in Japan under the brand name Alesion® and most often is used due to its antihistaminic effects.

For epinastine the oral application route (tablets, syrups etc.) is preferred and to mask the bitter taste sweetening agents may be added to the formulations.

Another interesting pharmacologically active substance is quinine. Quinine is a quinoline - alkaloid and thus again an amino base. Quinine has been and is still used to treat malaria, it has antipyretic, analgesic, muscle-relaxing properties and also can be used to induce labour-pains (childrenbirth). It is well known that quinine is bitter tasting and therefore it is even used as additive for some refreshing drinks.
Due to its bitter tasting properties it is also used as reference substance to estimate the bitterness of pharmaceuticals.

### Description of the Invention

Surprisingly it was found that the bitter taste of pharmacologically active substances like epinastine and quinine may be masked by sodium lauryl sulfate.

Sodium lauryl sulfate (SLS), which is also known as sodium dodecyl sulfate (SDS) is an anionic surfactant.

The objective of the present invention is to create not bitter-tasting pharmaceutical formulations of active ingredients with a bitter taste.

In the content of the present invention the bitterness tasting agents, which are subject of the present invention, shall not be mixed up with f.e. sweetening agents which also may interfere the bitter taste of the bitter tasting substances.

### Description of the present invention in detail

Subject of the present invention are pharmaceutical preparations which comprise a bitter tasting active substance and to mask said bitter taste by the addition of ionic surfactants to the preparation.

Surfactants are compounds with lyophobic and lyophibic properties which may be used as tensids. They easily interfere with the surface of chemical particles in particular if the particles and the surfactant are mixed together in a liquid. In the special case these lyophilc and lyophobic properties of the surfactant are seen with respect to water one also speaks of hydrophobic and hydrophilic properties. Surfactants may be an interface between such a liquid, f.e. water or an alcohol like ethanol, and a particle that shall be dissolved therein or suspended therein.

Often Surfactants have a lipophilic and a hydrophilic part on the molecular level. The lipophilic part often is an alkyl residue which is free from polar chemical groups such as -O-, -OH, -COOH, -OSO₃H₂, -amino, -alkylamine or -alkyldiamine. Such groups form the hydrophilc part of the molecule.
Surfactants bearing an acid group are called "anionic" if they are in the form of their salts.
Surfactants bearing an basic group are called "cationic" if they are in the form of their salts.
The surfactants preferably are used in the form of their salts. In the context of the present invention water soluble salts of the surfactants are preferred.

In the context of the present invention under the term "salt of an anionic surfactant" are meant surfactants with an anionic group which forms a salt with an alkali-ion, an earthalkali-ion, ammonium or a lower alkly mono- di- or triamine. Under the term "lower alkyl" are meant C₁ to C₆-alkyls in particular C₁- to C₄-alkyls, preferably methyl, ethyl, propyl.

As anionic surfactants are preferred pharmacologically acceptable salts of fatty acids, fatty alcohol-phosphates and fatty alcohol-sulfates. Preferred fatty acids are C₈ to C₂₁ alkane-acids. More preferred are C₁₀- to C₁₈- alkane-acids. The alkane acids may be saturated or insatured. Preferred are saturated ones. Under theses acids are preferred: laurylic acid (saturated C₁₂), myrisitinc acid (saturated C₁₄), palmitinic acid (saturated C₁₆), palmitoleinic acid (9-hexadecenic acid), oleic acid (cis-9-ocatadecenic acid), stearic acid (saturated C₁₈), linolic acid (Z,Z, 9-12 octadecadienic acid), linolenic aicd (Z,Z,Z, 9-12-15-octadecadienic acid), arachidonic acid (5-8-11-14-eicosatetraenic acid). Most preferred are the C₁₀- to C₁₄- alkane-acids, in particular laurylic acid (saturated C₁₂) and myrisitinc acid (saturated C₁₄).

Among the fatty alcohol-phosphates the following are preferred:
CH₃-(CH₂)ₙ-OPO3⁻ with n being an integer between 9 and 20, preferably between 9 and 17 and more preferably between 9 and 13.

Among the fatty alcohol-sulfates the following are preferred:
CH₃-(CH₂)ₙ-OSO3⁻ with n being an integer between 9 and 20, preferably between 9 and 17 and more preferably between 9 and 13.
The most preferred fatty alcohol-sulfate is lauryl sulfate (n= 11).

Fatty alcohol-sulfates are preferred as anionic surfactants.

Theses acids are used in form of their pharmaceutical acceptable salts as explained above. In particular preferred are the alkali salts and among theses the lithium-, sodium, potassium- salts. Most preferred are the sodium salts.

The most preferred bitter taste masking agent is the anionic surfactant sodium lauryl sulfate.

Examples of cationic surfactants are CH₃-(CH₂)ₙ-NR₁R₂ with n being an integer between 9 and 20, preferably between 9 and 17 and more preferably between 9 and 13; R₁ and R₂ being H or independently of each other C₁ to C₆-alkyls in particular C₁- to C₄-alkyls, preferably methyl, ethyl, propyl.

Theses bases are used in form of their pharmaceutical acceptable salts as explained above. In particular preferred are salts thereof with acids such as hydrochloric acid, sulfuric acid, hydrobromic acid, H₃PO₄, citiric acid, fumaric acid, succinic acid, tartaric acid, acetic acid.

The choice whether an anionic or an cationic surfactant is to be used depends on the chemical nature of the bitter tasting active ingredient. If this ingredient is basic in nature or the salt of a base, an anionic surfactant is used. If the bitter tasting ingredient is acidic in nature or a salt of an acid a cationic surfactant is used.

The amount of the ionic surfactant depends on the nature of it as well as on the amount of the bitter tasting ingredient.. The amount must be enough to mask the bitter taste and be less to go beyond the pharmacologically acceptable dosage. The amount of the surfactant may be between 0.01%w/w and 99.9% w/w, preferred are amounts between 0. 1%w/w and 60% w/w, more preferred are amounts between 0,5%w/w and 30% w/w. If not indicated otherwise w/w stands for weight per weight of the formulation.

In the case of sodium lauryl sulfate the preferred amount is between 0,1% and 50% weight per weight of bitter tasting ingredient, preferably the amount is not less than 10% weight per weight of the bitter tasting ingredient. The maximum amount per day is 300 mg. For example, if the amount of the ingredient is about 0.1% weight per weight of the formulation, the amount of SLS may be between 0.01%w/w and 99.9% weight per weight of the formulation.

In the context of the present invention, as bitter tasting active substance basic substances are preferred. More preferred are such organic pharmacologically active substances having an amino group. The amino group may be a primary, a secondary, a tertiary or a quartery amino function. Among such substances are epinastine and quinine. Both substances my be used in the form of the free bases, the hydrochloride, the sulfates and so on.

As outlined above epinastine is preferred in particular.

Pharmaceutical formulations comprising the same may be applied in any of the indications mentioned above (see also paragraph state of the art).

In preferred formulations the amount of epinastine is between 0,05% w/w and 60% w/w, more preferred is 0.5 % w/w and 30 %w/w.

In preferred formulations comprising quinine the amount of it is between 1% w/w and 80% w/w, more preferred is between 10 % w/w and 50 %w/w.

Among theses ingredients the inventive formulations may comprise other pharmaceutically acceptable adjuvants and additives.

The formulation may be in the form of a tablet , a syrup, a solution, a suspension or any other formulation which may be applied orally.

In the case of liquid formulations water, water-ethanol-mixtures or ethanol as liquid is preferred. In particular preferred is water. The invention also may be used in aerosol formulations which are to be inhaled into the lungs. Among such formulations are formulations with water, water-ethanol-mixtures or ethanol as liquid medium or propellant driven ones.

As adjuvans or additive the formulations may comprise sweetenig agents such as saccharin sodium, erithritol, aspartame, sugars and suger-derived polyols such as sucrose, D-sorbitol, glycerin and D-mannitol, glycyrrhizinates.

Other kinds of additives are pH-adjusting agents to adjust the pH of the resulted liquid to a value of preferably between 5 and 8, preferably 6 and 7. Among those agents are citric-acid, succinic acid, tartaric acid, acetic acid, citrates, acetates, vitamin C, hydrochloric acid, carbonates, phosphates, disodium phosphate, monosodium phosphate, sodium-, calcium-, potassium- and/or magnesium- hydroxide. Preferred are buffer substances like disodium phosphate.

Further commonly other used additives can optionally be added, too.
Among these are
- binding agents such as hydroxypropylcellulose, methylcellulose, hydroxypropylmethylcellulose, hydroxyethylcellulose, starch, dextrin, gelatine and polyvinylpyrrolidone, preferably hydroxypropylcellulose,
- flow agents such as hydrated silicon dioxide, light anhydrous silicic acid,
- odorizors such as sunfix orange No.22734 (commercial name, sunfix orange which is preferred contains orange flavour (30%w/w), acacia (30%w/w) and dextrin(40%w/w)), orange oil, mentha oil, eucalyputus strawberry flavour, vanilla flavour, yoghurt flavour and other flavours known in the art,
- colour pigments such as food yellow No.5, also being known as sunset yellow FCF (disodium salt of 6-hydroxyl-5-(4-sulfophenylazo)-2-naphthalenesulfonic acid), the latter being preferred, ferric oxide, and other food colour pigments, f.e. the ones known in Japan as food red No.3, 102,105 and 106, food yellow No.4 and other food colours known in the art, and/or
- preservatives such as benzoic acid, salts thereof, preferably sodium benzoate, paraoxybenzoic acids, salts thereof and other known preservatives, whereby sodium benzoate is preferred and
- effervescent agents such as bicarbonate.

Another aspect of the present invention refers to new salts of basic organic pharmacologically active ingredients and anionic surfactants, in particular as described above and salts of acidic organic pharmacologically active ingredients and cationic surfactants as described above. Such salts can be used in stead of mixing basic organic pharmacologically active ingredients or their salts with anionic surfactants or instead of mixing acidic organic pharmacologically active ingredients with cationic surfactants. The advantage of such a salt is that the active ingredient and the surfactant represent one chemical entity while the taste masking effect of the surfactant should be maintained.

Among theses new salts are epinastine lauryl sulfate and quinine lauryl sulfate.

The present invention relates also to a powder-like formulation (dry syrup) of an anionic surfactant as described above, preferably SLS and epinastine, either in enantiomeric, racemic form or a salt thereof, which is mixed with water prior to use. To do so it is required that the powder is dissolved in water very quickly.

In the context of the present invention the term "powder" is used simultaneously with the term "dry syrup" which in turn stands for an essentially water-free admixture of the active form of epinastine, preferably epinastine-hydrochloride, and pharmaceutically acceptable additives and adjuvans necessary to form an aqueous, sweet tasting formulation of the active substance when mixed with water.

Optionally, the formulation may comprise in addition sweetening agents for to mask a quick-acting and a lasting bitter taste caused by epinastine. For to mask the quick-acting bitter taste saccharin sodium, erithritol and/or aspartame turned out to be effective. Another suitable group of compounds comprises sugars and suger-derived polyols such as sucrose, D-sorbitol, glycerin and D-mannitol. Preferably, the formulation comprises saccharin sodium, erithritol and/or aspartame. Most preferred is the combination of saccharin sodium, erithritol and aspartame.

The amount of the these sweetening agents to mask the quick-acting bitterness depends of the agent used.
In case of saccharin sodium, it is between 0.1 % w/w and 2.0 % w/w of the powder formulation. Preferably the amount is 0.8. %

In case of erithritol it is between 50 % w/w and 95 % w/w of the powder formulation.
Preferably the amount is 75 to 80 % w/w, most preferred it is 80% w/w.

And in case of aspartame it is between 1 % w/w and 30 % w/w of the powder formulation. Preferably the amount is 5 to 15% w/w, most preferred it is 10%.

For masking the lasting bitterness glycyrrhizinates were found to be highly effective. Among them glycyrrhizinic acid and/or monoammonium glycyrrhizinate are the preferred ones. The most preferred one is monoammonium glycyrrhizinate.

The amount of monoammonium glycyrrhizinate in the powder formulation is 0.1 % w/w and 3.0 % w/w of the powder formulation. More preferred are 0.1 to 1% w/w and most preferred is 0.6%.

The formulation further may comprise adjuvans, among which are for example pH-adjusting agents and other agents as listed above.

The amount of epinastine or its salt is between 0.5 % w/w and 5 % w/w of the powder formulation. More Preferred is an amount of between 0,5 % w/w and 2 % w/w, the most preferred amount is 1 %.

The powder formulation preferably does not contain an active substance which is not epinastine or a pharmaceutically acceptable salt thereof.

The active substance, preferably epinastine-hydrochloride and all the additives are mixed to a powder and than the powder is mixed with water to preferably obtain a liquid formulation. Although the liquid formulation may either be a solution, suspension or a colloid, the preferred liquid formulation is a transparent and clear aqueous solution.

Alternatively to powders, tablets such as effervescent tablets may be used. The ingredients may be the same.

It is preferred to mix the powder formulation or the alternative thereof with water to obtain a liquid having a concentration of between 250mg per 5-50ml and 2000mg per 10-100 ml , preferably the amount is between 50 mg per 10 ml and 2000mg per 10ml.
If epinastine-hydrochlorid is used, the amount in the context of the present invention is (about) the same as for the free base.

In order to ensure that the patient easily can prepare the liquid formulation the inventive powder formulation (dry syrup) or the alternative thereof can be delivered in certain packages. In these packages the water and the inventive powder formulation are stored separately from each other. The package further allows the both components to mix in an easy way.

As a consequence thereof the present invention also relates to a kit comprising two components, a) the inventive powder formulation and b) water, both components separated from each other.

In the state of the art several bottles having special caps are known to solve this issue. Most often in such packages the liquid solvent can be stored in a bottle of glass, plastic, metal and so on while the cap for closing the bottle comprises a chamber to take the dry powder formulation. Prior to use the patient can take of the powder of the cap and mix it with the water in the bottle. This mixing process can either be done consciously, meaning the patient actively takes the powder and puts it into the water. In other embodiments the patient can initiate the mixing process in a more automatic way by for example just screwing, pressing, shaking the cap or the bottle in order to put away a barrier in the chamber containing the powder and by doing so allowing it to fall from the cap into the bottles.

Such packagings are disclosed for example in EP 0599189, EP 0344849, EP 0217425, EP 0093090, US 3802604 and others. Herewith, all of these devices are incorporated by reference. Other, similar devices might be used, too.
Besides, such package forms the dry syrup formulation can be stored in an aluminium or plastic-bag or in an aluminium or plastic bottle. The such stored powder then can be used with a pre-metered amount of water, stored in another package or the freshly filled drinking water is used.

Other package systems may be used, too.

In the context of the present invention the powder formulation can also be pressed to tablet to be dissolved in water, for example an effervescent tablet. In such an embodiment the tablet - just as the powder formulation - may additionally comprise an effervescent agent such as bicarbonate.

### Experimental

### Example 1:

0.1g g of epinastine-hydrochloride and 0.2 g of SLS were dissolved in 10g of water (sample 1).
0.1g g of epinastine-hydrochloride was dissolved in 10g of water (sample 2).
Sample2 (without SLS) was obviously bitterer than sample 1(with SLS). SLS significantly suppresses the bitterness of epinastine-hydrochloride in water.

### Example 2:

0.1g of quinine hydrochloride and 0.3 g of SLS were dissolved in 10 g of water (Sample 3)
0.1g of quinine hydrochloride was dissolved in 10 g of water (Sample 4)

Sample 4 (without SLS) was obviously bitterer than sample 3 (with SLS). SLS significantly suppresses the bitterness of quinine hydrochloride in water.

### Example 3:bitterness-masked dry syrup

Manufacturing procedure of the bitterness-masked dry syrup is as follows.
1. All ingredients of the epinastine-hydrochloride dry syrup except SLS are granulated (sample 5).
2. Spray a solution of SLS in water (5 % w/w) to the dry syrup in the fluidized bed granulator. (samples 6-8)
3. Compare the taste of sample 5 with samples 6 to 8.

**Table 1**

| formulation | | | | |
|---|---|---|---|---|
| | Sample 5 | Sample 6 | Sample 7 | Sample 8 |
| Epinastine-hydrochloride | 10 mg | 10 mg | 10 mg | 10 mg |
| Erythriol | 850 mg | 850 mg | 850 mg | 850 mg |
| Aspartame | 120 mg | 120 mg | 120 mg | 120 mg |
| Hydroxypopyl-cellulose | 20 mg | 20 mg | 20 mg | 20 mg |
| SLS | 0 mg | 10 mg | 20 mg | 30 mg |
| total | 1000 mg | 1010 mg | 1020 mg | 1030 mg |

### Results

Order of samples in bitterness corresponds to the amount of added SLS.
Sample 8 (SLS 3%) is the best taste among them. Comparing sample 5 with sample 8: In a test 11 persons out of 13 answered "sample 5
(SLS 0%) is obviously bitterer than Sample 8 (SLS 3%)"

### Example 4

New dry and aqueous epinastine- Syrup-Formulation 0.5%.

| | | | | | | | |
|---|---|---|---|---|---|---|---|
| Powder-Formulation No. | 1 | 2 | 3 | 4 | 5 | 6 | 7 |
| epinastine HCl [mg] | 5 | 5 | 5 | 5 | 5 | 5 | 5 |
| SLS [mg] | 10 | 10 | 10 | 10 | 10 | 10 | 10 |
| glycerin [mg] | 0 | 0 | 0 | 0 | 0 | 0 | 350 |
| sucrose [mg] | 0 | 0 | 0 | 0 | 0 | 540 | 610 |
| anhydr. citric acid [mg] | 0 | 0 | 0 | 0 | 0 | 25 | 0 |
| monoammonium glycyrrhizinate [mg] | 7 | 0 | 6 | 6 | 6 | 0 | 0 |
| saccharin sodium [mg] | 0 | 6 | 4 | 0 | 5 | 5 | 0 |
| sodium fumarate [mg] | 10 | 10 | 0 | 10 | 5 | 0 | 10 |
| disodium phosphate [mg] | 0 | 0 | 0 | 3 | 15 | 10 | 0 |
| erithritol [mg] | 918 | 919 | 925 | 916 | 904 | 380 | 0 |
| aspartame [mg] | 50 | 50 | 50 | 50 | 50 | 25 | 0 |
| sodium benzoate [mg] | 0 | 0 | 0 | 0 | 0 | 0 | 15 |

New dry and aqueous epinastine-Syrup-Formulation 1.0%

| | | | | | | | |
|---|---|---|---|---|---|---|---|
| Powder-Formulation No. | 8 | 9 | 10 | 11 | 12 | 13 | 14 |
| epinastine hydrochloride [mg] | 10 | 10 | 10 | 10 | 10 | 10 | 10 |
| SLS [mg] | 20 | 20 | 20 | 20 | 20 | 20 | 20 |
| glycerin [mg] | 0 | 0 | 0 | 0 | 0 | 0 | 350 |
| sucrose [mg] | 0 | 0 | 0 | 0 | 0 | 522 | 405 |
| anhydrous citric acid [mg] | 0 | 0 | 0 | 0 | 0 | 25 | 0 |
| monoammonium. glycyrrhizinate [mg] | 10 | 0 | 10 | 6 | 6 | 0 | 0 |
| saccharin sodium [mg] | 0 | 10 | 5 | 0 | 8 | 0 | 0 |
| sodium fumarate [mg] | 20 | 10 | 0 | 15 | 5 | 0 | 10 |
| disodium phosphate [mg] | 0 | 0 | 0 | 10 | 15 | 0 | 0 |
| erithritol [mg] | 859 | 849 | 854 | 838 | 835 | 397 | 200 |
| aspartame [mg] | 80 | 80 | 80 | 80 | 80 | 25 | 0 |
| hydroxypropylcellulose [mg] | 0 | 20 | 20 | 20 | 20 | 0 | 0 |
| hydrated silicon dioxide [mg] | 1 | 1 | 1 | 1 | 1 | 1 | 0 |
| Odorizor* [mg] | 0 | (1) | (1) | (1) | (1) | (1) | 0 |
| food yellow No.5** [mg] | 0 | (0.05) | (0.05) | (0.05) | (0.05) | (0.05) | 0 |
| sodium benzoate [mg] | 0 | 0 | 0 | 0 | 0 | 0 | 5 |

| | | | | | | | |
|---|---|---|---|---|---|---|---|
| *sunfix orange No.22734 (commercial name) containing orange flavor (30%w/w), acacia (30%w/w) and dextrin (40%w/w). | | | | | | | |
| *** Another name of Food yellow no.5 is sunset yellow FCF (disodium salt of 6-hydroxyl-5-(4-sulfophenylazo)-2-naphthalenesulfonic acid.* | | | | | | | |

## Claims

1. Pharmaceutical powder formulation comprising a) an active agent based on epinastine and/or an acid addition salt thereof, preferably the hydrochloride, and b) sodium lauryl sulfate, and c) optionally further pharmaceutically acceptable adjuvans, like sweetening agents and/or flavourings.

2. Pharmaceutical powder formulation according to claim 1, **characterised in that** the formulations comprises two kinds of sweetening agents, one for to mask the quick-acting bitterness of epinastine which is selected from saccharin sodium, erithritol, aspartame and/or sugars or other polyols such as sucrose, glycerin, D-sorbitol and D-mannitol and another one for to mask a lasting bitterness of epinastine.

3. Pharmaceutical powder formulation according to claim 1 or 2, **characterised in that** the formulations comprises two kinds of sweetening agents, one for to mast the quick-acting bitterness of epinastine and one for to mask the long-acting bitterness which is selected from a glycyrrhizinate or glycyrrhinic acid, preferably selected from mono-, di-ammonium glycyrrhizinate, di-, tri-sodium glycyrrhizinate and/or dipotassium glycyrrhizinate, most preferably monoammonium glycyrrhizinate.

4. Pharmaceutical powder formulation according to any of claims 1 to 3, **characterised in that** the formulation further comprises a pH-adjusting agent selected from the group of citric-acid, citrates, succinic acid, tartaric acid, acetic acid, acetates, vitamine C, hydrochloric acid, carbonates, sodium-, calcium-, potassium- and/or magnesium- hydroxide, phosphates, preferably disodiumphosphate and/or monosodium phosphate, most preferably monosodium phosphate.

5. Pharmaceutical powder formulation according to any of claims 1 to 4, **characterised in that** the formulation further comprises at least one adjuvans selected from the group of odorizers, colour pigments, preservatives, flow agents and/or binding agents.

6. Pharmaceutical powder formulation according to any of claims 1 to 5, **characterised in that** the formulation comprises epinastine-hydrochloride in an amount of between 0.5% w/w and 5 %w/w, sodium lauryl sulfate in an amount of between 0.05 % w/w and 50 %w/w, preferably between 10% and 50% weight per weight of epinastine, saccharin sodium in an amount of between 0.1 % w/w and 2.0 % w/w, erithritol in an amount of between 50 % w/w and 95 % w/w, aspartame in an amount of between 1 % w/w and 30 % w/w, monoammonium glycyrrhizinate in an amount of 0.1 % w/w and 3.0 % w/w and optionally disodiumphosphate, sodium fumarate, hydroxypropylcellulose, hydrated silicon dioxide, orange flavor, acacia, dextrin and /or disodium salt of 6-hydroxyl-5-(4-sulfophenylazo)-2-naphthalenesulfonic acid.

7. Pharmaceutical powder formulation according to any of claims 1 to 6, **characterised in that** the formulation comprises epinastine hydrochloride, sodium lauryl sulfate, monoammonium glycyrrhizinate, saccharin sodium, sodium fumarate, erithritol, aspartame, hydroxypropylcellulose, hydrated silicon dioxide, orange flavour, acacia, dextrin and disodium salt of 6-hydroxyl-5-(4-sulfophenylazo)-2-naphthalenesulfonic acid.

8. Pharmaceutical powder formulation according to any of claims 1 to 8 **characterised in that** the formulation comprises an effervescent agent.

9. Pharmaceutical tablet, comprising the ingredients of the powder formulation according to any of claims 1 to 8.

10. Liquid formulation available by mixing any of the powder formulations of claims 1 to 8 or the tablet of claims 9 with water.

11. Liquid formulation according to claim 10, **characterised in that** the formulation is a solution.

12. Liquid formulation according to claim 10, **characterised in that** the formulation is a dispersion.

13. Liquid formulation according to claim 11 or 12, **characterised in that** the formulation comprises a pH-adjusting agent according to claim 6 in an amount to adjust the pH to between 5 and 8, preferably to between 6 and 7.

14. Liquid formulation according to any of claims 11 to 13, **characterised by** an amount of epinastine of between 0.09 mg and 35 mg per 10 ml, preferably of between 0.4 mg and 18 mg per 10 ml.

15. Liquid formulation according to any of claims 11 to 13, **characterised by** an amount of epinastine-hydrochloride of between 0.1 mg and 40 mg per 10 ml, preferably of between 0.5 mg and 20 mg per 10 ml.

16. Liquid formulation according to any of claims 11 to 15, **characterised in that** 250 mg to 2000 mg of a powder formulation according to any of claims 1 to 9 or the tablet of claim 9 are mixed with 10 to 100 ml of water.

17. Pharmaceutical preparation comprising a bitter tasting organic pharmacologically active ingredient and a ionic surfactant masking the bitterness.

18. Pharmaceutical preparation according to claim 17, **characterised in that** the bitter tasting organic pharmacologically active ingredient is a base and the ionic surfactant is an anionic surfactant or the bitter tasting organic pharmacologically active ingredient is an acid and the ionic surfactant is a cationic surfactant.

19. Pharmaceutical preparation according to claim 17 or 18, **characterised in that** the bitter tasting organic pharmacologically active ingredient is a base and the anionic surfactant is a salt of a fatty acid, a fatty alcohol-phosphate or a fatty alcohol-sulfate each of which having an alkyl chain of eight to twenty-one carbon atoms.

20. Pharmaceutical preparation according to claim 17, 18 or 19, **characterised in that** the bitter tasting organic pharmacologically active ingredient is a base and the anionic surfactant is sodium lauryl sulfate.

21. Pharmaceutical preparation according to claim 17 or 18, **characterised in that** the bitter tasting organic pharmacologically active ingredient is an acid the surfactant is a cationic surfactant which is selected from the salt of a fatty alcohol-amine having an alkyl chain of eight to twenty-one carbon atoms.

22. Pharmaceutical preparation according to any of claims 17 to 20, **characterised in that** the bitter tasting organic pharmacologically active ingredient is epinastine or a pharmacologically acceptable salt thereof or quinine or pharmacologically acceptable salt thereof.

23. Pharmaceutical preparation according to any of claims 17 to 22, **characterised in that** the amount of the bitter tasting organic pharmacologically active ingredient is between 0.01 % w/w and 80 % w/w.

24. Pharmaceutical preparation according to any of claims 17 to 20, and 22 to 23 **characterised in that** the amount of sodium lauryl sulfate is between 0.01 % w/w and 99.9 % w/w.

25. Salt of a basic organic pharmacologically active ingredient and an anionic surfactant and salts of acidic organic pharmacologically active ingredient and a cationic surfactant.

26. Salt according to claim 25, **characterised in that** the organic pharmacologically active ingredient is a base or a salt thereof and the surfactant is an anionic one which is selected from a) fatty acids such as C₈ to C₂₁ alkane-acids, b) fatty alcohol-phosphates such as CH₃-(CH₂)ₙ-OPO3⁻ with n being an integer between 9 and 20, preferably between 9 and 17 and more preferably between 9 and 13 or c) fatty alcohol-sulfates such as CH₃-(CH₂)ₙ-OSO3⁻ with n being an integer between 9 and 20, preferably between 9 and 17 and more preferably between 9 and 13.

27. Salt according to claim 25, **characterised in that** the organic pharmacologically active ingredient is an acid or a salt thereof and the surfactant a cationic one and it is selected from CH₃-(CH₂)ₙ-NR₁R₂ with n being an integer between 9 and 20, preferably between 9 and 17 and more preferably between 9 and 13; R₁ and R₂ being H or independently of each other C₁ to C₆-alkyls in particular C₁- to C₄-alkyls, preferably methyl, ethyl, propyl.

28. Salt according to claim 25 or 26, **characterised in that** the active ingredient is epinastine or a pharmacologically acceptable salt thereof or quinine or a pharmacologically acceptable salt thereof.

29. Salt according to claim 28, **characterised in that** the surfactant is sodium lauryl sulfate.

30. Package comprising a kit of a powder formulation according to any of claims 1 to 8 or the tablet of claim 9 and separated thereof water to obtain a liquid formulation according to any of claims 10 to 16 if the powder and the water are mixed together.

31. Package according to claim 30, **characterised in that** the package is a bottle with a cap comprising a chamber, that can be opened easily in a manual way or by screwing or pressing the cap onto the bottle, the chamber containing a powder formulation according to any of claims 1 to 8 or the tablet of claim 9 and the bottle comprising the water.

32. Use of a powder formulation according to any of claims 1 to 8 or use of the tablet of claim 9 to mix with water to obtain a formulation according to any of claims 10 to 16.

33. Use of a formulation according to any of claims 1 to 16 for to manufacture a medical preparation for to treat allergies, pain, in particular chronic pain and inflammation caused pain, migraine, asthma, rhinitis, conjunctivitis and/or bronchitis, preferably for to treat allergies.

34. Method of treating allergies, pain, in particular chronic pain and inflammation caused pain, migraine, asthma, rhinitis, conjunctivitis and/or bronchitis, preferably for to treating allergies **characterised by** mixing a powder formulation according to any of claim 1 to 8 or the tablet of claim 9 with water to obtain a liquid formulation according to any of claims 10 to 16 and to apply this formulation to a patient.

35. Process for making a powder formulation according to any of claims 1 to 8 by mixing the different components together.

36. Process for making a tablet according to claim 9 by mixing the different components together and subsequent pressing thereof.

37. Process for making a liquid formulation according to any of claims 10 to 16 by mixing the powder formulation according to any of claims 1 to 8 or the tablet according to claim 9 with water.
